# EUROPEAN PATENT APPLICATION

(11) **EP 3 939 502 A1**
(43) Date of publication of application: **19.01.2022**
(21) Application number: 20769440.7
(22) Date of filing: 11.03.2020
(51) Int. Cl.: A61B 5/026, A61B 5/16

(54) **SLEEP ESTIMATION SYSTEM, SLEEP ESTIMATION DEVICE, AND SLEEP ESTIMATION METHOD**

(30) Priority: 11.03.2019 JP 2019043448
(71) Applicant: Kyocera Corporation, Kyoto-shi, Kyoto 612-8501 (JP)
(72) Inventor: WATANABE, Takahiro, Kyoto-shi, Kyoto 612-8501 (JP)
(74) Representative: TBK
(86) International application number: PCT/JP2020/010619
(87) International publication number: WO 2020/184627

(57) **Abstract**

A sleep estimation system (1) according to an embodiment includes: an input device (14, 16) configured to receive blood flow data on a user; and a controller (11) configured to calculate output data (19) indicating a sleep state of the user on the basis of the blood flow data, in which the controller includes an approximator (17) capable of calculating the output data on the basis of the blood flow data.

## Description

### Technical Field

The present disclosure relates to estimation of sleep.

### Background Art

Patent Document 1 describes a technology for detecting a sleep state.

### Citation List

### Patent Document

Patent Document 1: JP 2018-161432 A

### Summary

### Technical Problem

There has been a demand for a way to estimate a sleep state with high accuracy.

### Solution to Problem

A sleep estimation system according to an embodiment includes: an input device configured to receive blood flow data on a user; and a controller configured to calculate output data indicating a sleep state of the user from the blood flow data, in which the controller includes an approximator capable of calculating the output data from the blood flow data.

A sleep estimation program according to an embodiment is a sleep estimation program for causing a computer to function as the controller included in the sleep estimation system described above.

A sleep estimation device according to an embodiment includes: an input unit configured to receive blood flow data on a user; and a control unit configured to form an approximator including an input layer configured to receive the blood flow data, a hidden layer configured to perform calculation based on a learned parameter to an output from the input layer, and an output layer configured to output a calculation result of the hidden layer as a sleep state of the user.

A method of estimating sleep according to an embodiment is a method of estimating sleep using an approximator including: an input layer configured to receive blood flow data on a user; a hidden layer configured to perform calculation based on a learned parameter on an output from the input layer; and an output layer configured to output a calculation result of the hidden layer as a sleep state of the user, the method including the steps of: transmitting, by the input layer, the blood flow data inputted into the approximator to the hidden layer; performing, by the hidden layer, calculation based on a learned parameter on the blood flow data; and outputting, by the output layer, an estimated sleep state of the user on a basis of a calculation result of the hidden layer.

### Advantageous Effects of Invention

It is possible to improve the accuracy of estimation of a sleep state.

### Brief Description of Drawings

FIG. 1 is a diagram schematically illustrating a configuration of a sleep estimation system according to an embodiment.
FIG. 2 is a diagram for explaining an operation of the sleep estimation system in FIG. 1.
FIG. 3 is a diagram for explaining an operation of the sleep estimation system in FIG. 1.
FIG. 4 is a schematic diagram illustrating an example of a change in brain waves.
FIG. 5 is a diagram schematically illustrating a configuration of a sleep estimation system according to another embodiment.

### Description of Embodiments

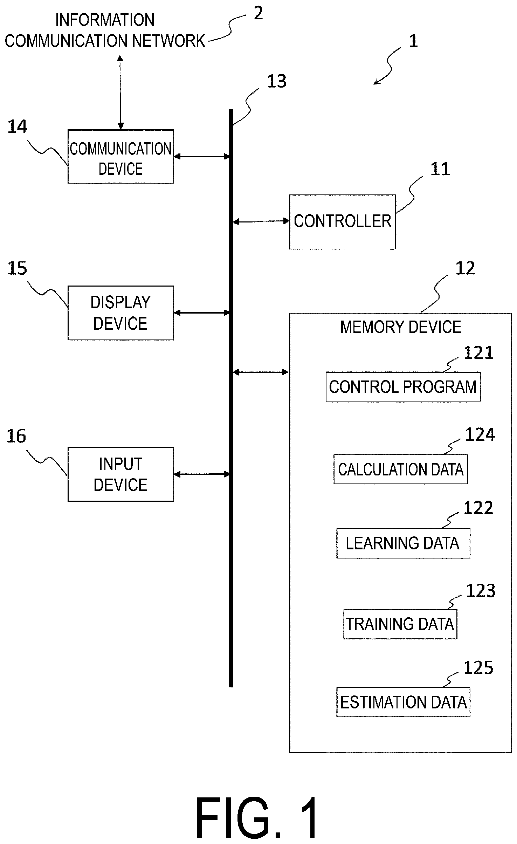

FIG. 1 is a schematic view illustrating the configuration of a sleep estimation system according to an embodiment.

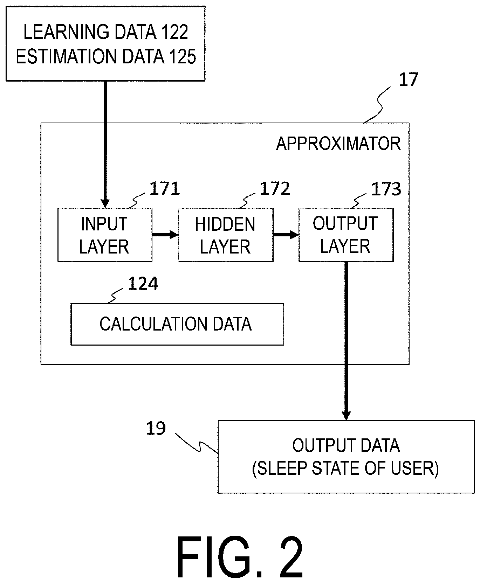

A sleep estimation system 1 can estimate a sleep state of a user on the basis of data related to blood flow of the user (blood flow data). Specifically, for example, the sleep estimation system 1 causes an approximator 17 illustrated in FIG. 2 to perform calculation on the basis of the inputted blood flow data, thereby making it possible to estimate a sleep state of the user. Note that the approximator 17 may include a so-called neural network. Note that the neural network represents a mathematical model that simulates the neurons of the cerebral nervous system of a human. The approximator 17 may include a learned mathematical model (for example, arithmetic expression) as described below.

The sleep estimation system 1 includes a controller 11, a memory device 12, and a bus (bus) 13. The various devices that constitute the sleep estimation system 1, such as the controller 11 and the memory device 12, are connected electrically or optically to each other through the bus (bus) 13, and can communicate with each other.

The controller 11 can collectively manage operation of the sleep estimation system 1 by controlling other constituent elements of the sleep estimation system 1. The controller 11 includes at least one processor to provide control and processing power used to perform various functions. According to various embodiments, the at least one processor may be implemented as a single integrated circuit (IC: Integrated Circuit) or a plurality of communicatively connected ICs and/or discrete circuits (Discrete Circuits). The at least one processor can be run in accordance with various known techniques.

The processor may include, for example, one or more circuits, units, or firmware (for example, a discrete logic component) configured to perform one or more data computation procedures or processes by executing an instruction, such as a program, stored in an associated memory, such as memory device 12. In addition, the processor may include one or more processors, controllers, microprocessors, micro-controllers, application specific integrated circuits (application specific IC: ASIC), digital-signal processing devices, programmable logic devices, and field-programmable gate arrays, or a combination of any of these devices or configurations, or a combination of other known devices or configurations. The controller 11 of the sleep estimation system 1 according to an embodiment includes, for example, a central processing unit (CPU).

The memory device 12 can store, for example, various information and programs to realize the functions of the sleep estimation system 1. Specifically, the memory device 12 can store a control program 121 serving as a program for controlling the sleep estimation system 1. The memory device 12 includes, for example, a non-transitory recording medium readable by the CPU of the controller 11 such as a read only memory (ROM) or a random access memory (RAM). The memory device 12 can be configured using a conventionally known technique.

The controller 11 can realize various functions by executing the control program 121 in the memory device 12. That is, the control program 121 includes a sleep estimation program for causing the computer to estimate a sleep state of a user. In the sleep estimation system 1 according to an embodiment, by causing the controller 11 to execute the control program 121 in the memory device 12, it is possible to form an approximator 17 that can estimate a sleep state of a user.

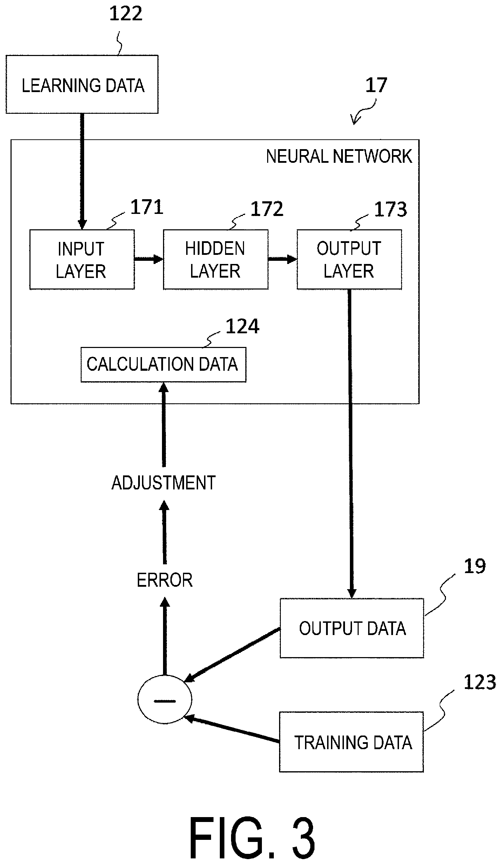

FIG. 2 is a schematic view illustrating an operation until the sleep estimation system illustrated in FIG. 1 estimates a sleep state of a user. In addition, FIG. 3 is a diagram used to explain one example of learning by the approximator 17.

Note that the controller 11 performs the operation until a sleep state of a user is estimated as illustrated in FIG. 2. Specifically, the controller 11 executes the control program 121 in the memory device 12 to form the approximator 17 that can estimate a sleep state of the user. In addition, the controller 11 calculates output data (for example, a sleep state of a user) from estimation data 125 (for example, blood flow data) of the user in accordance with the approximator 17, and outputs the data.

The approximator 17 includes, for example, an input layer 171, a hidden layer 172, an output layer 173, and a calculation data 124. The input layer 171 can transmit inputted data to the hidden layer 172. The hidden layer 172 can perform various operations on the data inputted from the input layer 171 on the basis of the calculation data 124. In addition, the hidden layer 172 can output a calculation result to the output layer 173. The output layer 173 can output, as output data 19, the calculation result inputted from the hidden layer 172. Note that, although not illustrated, each layer is made up of units in which signals are input and output.

The sleep estimation system 1 can improve the accuracy of a calculation result by causing the approximator 17 to learn. The learning by the approximator 17 refers to adjusting the strength of connection between units and the bias of the connection so that a correct calculation result is output from the output layer 173. Specifically, it refers to comparing the output data 19 based on the learning data 122 with the training data 123 serving as data of the correct answer prepared in advance, and then, adjusting the calculation data 124 so as to bring the output data 19 close to the training data 123. The sleep estimation system 1 according to an embodiment can improve the accuracy of estimation by adjusting the calculation data 124 through so-called supervised learning using the learning data 122 and the training data 123.

The learning data 122 is example data for causing the approximator 17 to learn. The learning data 122 includes information based on the metabolism of a human (metabolic information). Here, the metabolic information includes, for example, information related to blood flow, respiration, perspiration, body temperature, body movement, and the like.

The metabolic information may be acquired in advance using an arbitrary measurement device. Information related to the blood flow may be measured, for example, using a blood flow sensor such as a laser Doppler flowmeter, an ultrasound blood flowmeter, or a photoplethysmography meter. In addition, as for information related to respiration, the depth of breathing or the respiratory rate or the like may be acquired as a sound, for example, using a microphone, or the information related to respiration may be acquired as movement of a chest using an acceleration sensor. In addition, information related to perspiration may be acquired, for example, by attaching a piece of cotton to any part of the body and acquiring the amount or weight of perspiration absorbed as the amount of perspiration. Furthermore, information related to body temperature may be acquired, for example, by using a thermometer such as a thermistor, an infrared sensor, or a mercury thermometer. Information related to body movement may be measured, for example, by using an acceleration sensor or a pressure sensor. In addition, a combination of a plurality of various pieces of information measured as described above may be used as the metabolic information that the sleep estimation system 1 utilizes.

Note that the metabolic information that the sleep estimation system 1 utilizes may be information with a behavior that changes between during wakening and during sleep or information with a behavior that changes in accordance with the depth of sleep. In addition, the learning data 122 may be data in which the metabolic information is associated with personal information and lifestyle habits that may affect mechanisms of the cardiovascular system and include age, gender, height, body fat, drinking habits, and smoking habits.

A sleep measurement system 1 according to the present disclosure can use blood flow data as the learning data 122. The blood flow data may be data including various variables computed on the basis of blood flow. Specifically, for example, the blood flow data may be data related to at least one of the amount of blood flow, heart rate, heart beat interval, cardiac output, blood flow wave height, and the coefficient of variation of blood vessel motion (vasomotion).

The amount of blood flow represents the amount of blood flowing in a blood vessel per unit time. The heart rate represents the number of beats of the heart per unit time. The heart beat interval represents an interval between beats of the heart. The cardiac output represents the amount of blood delivered in one beat of the heart. The blood flow wave height represents a difference between the maximum value and the minimum value of the amount of blood flow in one beat of the heart. The vasomotion represents a contraction-expansion movement of the blood vessel that occurs spontaneously and rhythmically. The coefficient of variation of vasomotion represents a value indicating, as a variation, the change in the amount of blood flow occurring on the basis of the vasomotion.

The training data 123 is data on the correct answers associated with the learning data 122. The sleep estimation system 1 according to an embodiment can use, as the training data 123, data in which blood flow data of the learning data 122 is associated with the sleep state of a person from which this blood flow data is acquired. Specifically, the training data 123 includes, for example: data in which a change in the amount of blood flow is associated with a change in sleep state; data in which a change in the heart rate is associated with a change in sleep state; data in which a change in the heart beat interval is associated with a change in sleep state; data in which a change in cardiac output is associated with a change in sleep state; data in which a change of the wave height of blood flow is associated with a change in sleep state; or data in which a change in the coefficient of variation of vasomotion is associated with a change in sleep state. Note that, in a case where the training data 123 is acquired, the sleep state of a user may be measured using an arbitrary measurement device that can acquire the sleep state during acquisition of the learning data 122. For example, by measuring brain waves using an electroencephalograph during acquisition of the blood flow data using a blood flow sensor, it is possible to acquire data on the sleep state of a user that is associated with the blood flow data serving as the learning data 122.

The calculation data 124 includes data related to calculation by the approximator 17 and includes data including arithmetic expressions, constants, and variables of the arithmetic expressions such as biases and weights, and operators. In addition, the calculation data 124 includes, for example, a learned parameter such as a mathematical model. Note that, in the calculation data 124, the biases and weights define the strength of connection between individual units in the approximator 17. Thus, by adjusting the constants and variables such as the biases and weights through learning, the sleep estimation system 1 can adjust the calculation results of the approximator 17.

As for the method of adjusting the calculation data 124, for example, backpropagation, gradient descent, and the like may be employed. The calculation data 124 that has been adjusted through learning may be stored in the memory device 12 as the learned calculation data 124. Note that the adjustment method is not limited to the examples described above, and any method may be used, provided that the method can adjust the calculation data 124 so as to improve the accuracy of estimation of a sleep state through learning.

When performing learning, the learning data 122 is first inputted into the input layer 171. Then, in the hidden layer 172, calculation based on the calculation data 124 is performed on the learning data 122. Next, a calculation result of the hidden layer 172 is outputted from the output layer 173 as the output data 19. In addition, the approximator 17 compares the training data 123 and the output data 19 to adjust the calculation data 124 so as to reduce the error.

In this manner, the sleep estimation system 1 according to an embodiment can train the neural network by using the learning data 122, the training data 123, and the calculation data 124. Thus, the sleep estimation system 1 enables the accuracy of estimation of a sleep state to be improved.

In addition to the sleep estimation system 1, the learning of the approximator 17 may be performed by another device. In this case, the learned calculation data 124 generated by the other device is stored in the memory device 12 of the sleep estimation system 1. Furthermore, the memory device 12 does not need to hold the learning data 122 and the training data 123. The communication device 14 receives, through an information communication network 2, the learned calculation data 124 generated by the other device, and the controller 11 can cause the learned calculation data 124 received by the communication device 14 to be stored in the memory device 12. In addition, the learned calculation data 124 generated by the other device may be stored in a removable memory included in the memory device 12.

The sleep estimation system 1 according to an embodiment can estimate a sleep state of a user by using the learned approximator 17. Specifically, by performing calculation on the inputted estimation data 125 with the approximator 17, the sleep estimation system 1 can estimate a sleep state. In this case, the estimation data 125 is first inputted in the input layer 171. Next, in the hidden layer 172, calculation based on the calculation data 124 is performed on the estimation data 125. Then, the output data 19, which is a calculation result, is outputted from the output layer 173 as the estimated sleep state.

The estimation data 125 is data inputted into the learned approximator 17 and used to estimate a sleep state of a user. Note that the type of the learning data 122 may be the same type as the estimation data 125. That is, in a case where a sleep state of a user is desired to be estimated on the basis of information related to the blood flow, the estimation data 125 and the learning data 122 may be blood flow data. As an example, the metabolic information such as blood flow data is acquired using a measurement device that measures the metabolism of a user of which a sleep state is desired to be estimated, and the metabolic information acquired using this measurement device is inputted into the approximator 17 as the estimation data 125.

In the manner described above, the sleep estimation system 1 according to an embodiment can estimate a sleep state of a user on the basis of the estimation data 125.

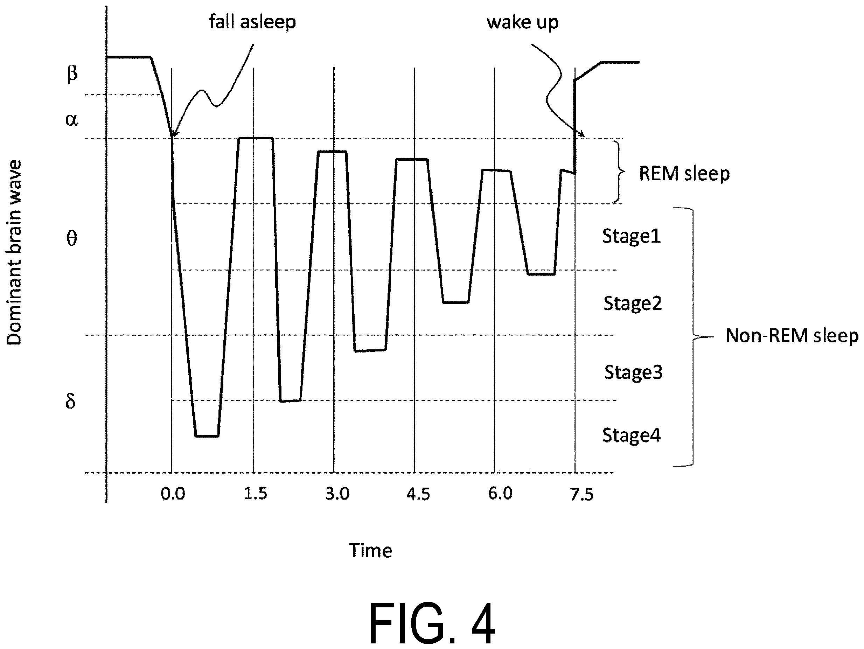

FIG. 4 is a schematic view illustrating an example of change in brain waves related to sleep.

The sleep state can be categorized, for example, on the basis of the brain waves. Specifically, a sleep state can be categorized into a state where a user is awake, a state where the user is in shallow sleep such as during rapid eye movement sleep (REM sleep), and a state where the user is in deep sleep such as during non-rapid eye movement sleep (Non-REM sleep). In addition, the non-REM sleep can be further categorized according to the depth of sleep. For example, the non-REM sleep can be categorized as stage 1, stage 2, stage 3, and stage 4 in order of decreasing sleep intensity.

The brain waves are divided into four categories, β wave, α wave, θ wave, and δ wave, in order from the longest wavelength. The β wave represents a brain wave with a frequency that falls, for example, in a range from 38 Hz to approximately 14 Hz. The α wave represents a brain wave with a frequency that falls, for example, in a range from 14 Hz to approximately 8 Hz. The θ wave represents a brain wave with a frequency that falls, for example, in a range from 8 Hz to approximately 4 Hz. The δ wave represents a brain wave with a frequency that falls, for example, in a range from 4 Hz to approximately 0.5 Hz.

A person is asleep in a case where the θ wave and the δ wave are dominant relative to the β wave and the α wave. Here, the expression "are dominant" means that the percentage of a certain wave is large in the measured brain waves. It is known that, during sleep, the dominant brain wave periodically changes in a range of from the θ wave to the δ wave (FIG. 4). In addition, in a case where the percentage of the θ wave included in the brain waves is less than a predetermined percentage, a person is in a state of REM sleep. In a case where the percentage of the θ wave is equal to or more than a predetermined percentage and in a case where the δ wave is dominant, a person is in a state of non-REM sleep (FIG. 4).

Note that REM sleep represents sleep involving rapid eye movement (Rapid Eye Movement: REM). CHECK Non-REM sleep represents sleep not involving rapid eye movement.

The sleep state has been estimated using the brain waves acquired with an electroencephalograph. However, highly specialized skills are necessary to handle an electroencephalograph and acquire the brain waves. In addition, attachment of an electroencephalograph is complicated. This makes it difficult for a user to acquire data about their brain waves in a simple manner, and difficult for the user to know their own sleep state in a simple manner.

In contrast, the sleep estimation system 1 can estimate a sleep state of a user on the basis of the blood flow data. No highly specialized skills are necessary to handle a device that measures blood flow and acquire the blood flow data, as compared with an electroencephalograph. In addition, it is easier to attach a device that measures the blood flow, as compared with an electroencephalograph. That is, a user can acquire their own blood flow data in a relatively simple manner. Thus, with the sleep estimation system 1, it is possible for a user to know their own sleep state in a simple manner.

Furthermore, for example, when waking up during deep sleep, a heavy feeling of sleepiness or drowsiness is more likely to occur immediately after waking up, causing a state (so-called sleep inertia) where the brain does not function well. On the other hand, the sleep inertia is less likely to occur if a relatively shallow sleep state such as stage 1 or 2 of non-REM sleep is set as the wake-up time. Thus, with the sleep estimation system 1, a user can know an optimum wake-up time, sleep cycles, and the like, which makes it possible to improve the usefulness of the system.

Since bodily functions of the human body are controlled by the brain during sleep, there is a correlation between a change in sleep state and a change in blood flow or vasomotion. Specifically, as sleep deepens, brain function decreases, which results in suppression of blood vessel activities such as beating of the heart or vasomotion as well as bodily functions. Therefore, as sleep becomes deeper, blood flow is reduced. This enables the sleep estimation system 1 according to an embodiment to estimate the sleep state of a user on the basis of blood flow data.

The amount of blood flow included in the blood flow data is an index including various elements related to blood flow such as heart rate, heart beat interval, cardiac output, blood flow wave height, and vasomotion. Thus, in a case of using data of the amount of blood flow as the learning data 122, the sleep estimation system 1 can cause the approximator 17 to learn on the basis of the various elements related to blood flow, which makes it possible to improve the usefulness of the system.

Furthermore, at the time of sleep onset, it is known that the activity of the parasympathetic nerve increases in a human body. Vasomotion is more likely to be susceptible to the influence of the effect of the parasympathetic nerve. Thus, the coefficient of variation of vasomotion is more likely to vary largely in association with a change in sleep state. In other words, a change in sleep state and a change in the coefficient of variation of vasomotion are more likely to exhibit a relatively high correlation. Thus, by using, as the learning data 122, data on the coefficient of variation of vasomotion included in the blood flow data, the sleep estimation system 1 can cause the approximator 17 to effectively learn the correlation between a sleep state and blood flow, which makes it possible to improve the accuracy of estimation.

Furthermore, the blood flow may be affected not only by a sleep state but also by the surrounding environment during sleep. For example, when a user is using an air conditioner during sleep, if the site where the blood flow data is acquired is cooled by the wind from the air conditioner, the amount of blood flow may be a value lower than expected. On the other hand, the heart rate is a value based on beating of the heart, and hence, a measurement error caused by the surrounding environment is likely to be relatively small. Thus, by using, as the learning data 122, data on the heart rate included in the blood flow data, the sleep estimation system 1 can improve the accuracy of estimation.

The sleep state that the sleep estimation system 1 can estimate includes, for example, the wakefulness, REM sleep, and non-REM sleep of a user. That is, the user can know a change in their sleep state. In addition, the sleep estimation system 1 can estimate the individual stages of the state of the non-REM sleep. Specifically, on the basis of the blood flow data, the sleep estimation system 1 can estimate which stage of the stages 1 to 4 the non-REM sleep is in. Thus, with the sleep estimation system 1, the user can know the optimum wake-up time, sleep cycles, and the like.

Furthermore, the learning of the approximator 17 may also be performed for each of the sleep states. For example, weighting may be performed on the calculation data 124 for each of the sleep states by using the learning data 122 and the training data 123 during waking; the learning data 122 and the training data 123 during REM sleep; and the learning data 122 and the training data 123 during non-REM sleep. Thus, it is possible to use the learning data 122 and the training data 123 each having a prominent feature for each of the sleep states, which makes it possible to cause the approximator 17 to efficiently learn.

The site where the blood flow data included in the learning data 122 is acquired may be, for example, an ear, a finger, a wrist, an arm, a forehead, a nose, or a neck. Furthermore, the blood flow data may be acquired, for example, at a concha auricula, an ear canal, an ear lobe, or a tragus among the parts of an ear. Thus, a user can select the site where the blood flow data is to be acquired, which improves the convenience of the sleep estimation system 1.

The configuration of the sleep estimation system 1 according to the embodiment described above is not limited to the examples described above. For example, the controller 11 may include a plurality of CPUs. In addition, the controller 11 may include at least one DSP. Furthermore, all the functions of the controller 11 or some of the functions of the controller 11 may be achieved with a hardware circuit that does not require any software to achieve the functions. Moreover, the memory device 12 may include a non-transitory recording medium readable by a computer and other than the ROM or RAM. The memory device 12 may include, for example, a compact hard disk drive, a solid state drive (SSD), and the like. Furthermore, the memory device 12 may be a memory such as a universal serial bus (USB) memory that can be attached to or detached from the sleep estimation system 1. After this, the memory that can be attached to or detached from the sleep estimation system 1 may be referred to as a "detachable memory".

Furthermore, the sleep estimation system 1 may further include a communication device 14 that can communicate with an arbitrary external electronic device, a display device 15 that can display the system of the sleep estimation system 1 and various types of upper tables such as sleep states of a user, and an input device 16 that can input various types of information and signals into the sleep estimation system 1. Note that the communication device 14, the display device 15, and the input device 16 may be electrically or optically connected to each other through the bus (bus) 13.

The communication device 14 according to an embodiment can be connected, through wired communication or wireless communication, to the information communication network 2 such as the Internet, which enables the sleep estimation system 1 and an external device to be connected to each other. That is, the communication device 14 can communicate with other devices such as a cloud server and a web server through the information communication network 2. In addition, the communication device 14 can input, into the controller 11, various types of information received from the information communication network 2. Furthermore, the communication device 14 can transmit information received from the controller 11 to the information communication network 2.

The display device 15 according to an embodiment can display various types of information such as a letter, a symbol, or a diagram through control by the controller 11. The display device 15 can be configured, for example, with a known technique such as a liquid crystal display or an organic EL display.

The input device 16 according to an embodiment can output, as a signal, data inputted from a user to the controller 11. The input device 16 may be an interface that can output a signal on the basis of operation by a user and includes, for example, a keyboard, a mouse, a touch panel, and the like. In addition, in a case of using a touch panel or other interfaces that can output a signal based on operation by a user and display various information, the display device 15 and the input device 16 may function as one unit. Thus, it is possible to improve the convenience of the sleep estimation system 1. The input device 16 may be configured with a known technique.

In addition, the learning data 122, the training data 123, the calculation data 124, and the estimation data 125 may be inputted into the controller 11 through the communication device 14 or the input device 16. These pieces of inputted data may be stored in the memory device 12. In addition, the training data 123 and the calculation data 124 may be stored in advance in the memory device 12. Furthermore, the memory device 12 may store, as supervised learning data, data in which the learning data 122 and the training data 123 are brought together.

In addition, the display device 15 may display the estimated sleep state of a user. Furthermore, the output data 19 outputted as the sleep state of a user may be used in other devices. In this case, the output data 19 may be outputted to the outside through the communication device 14 or the like.

### Other Embodiments

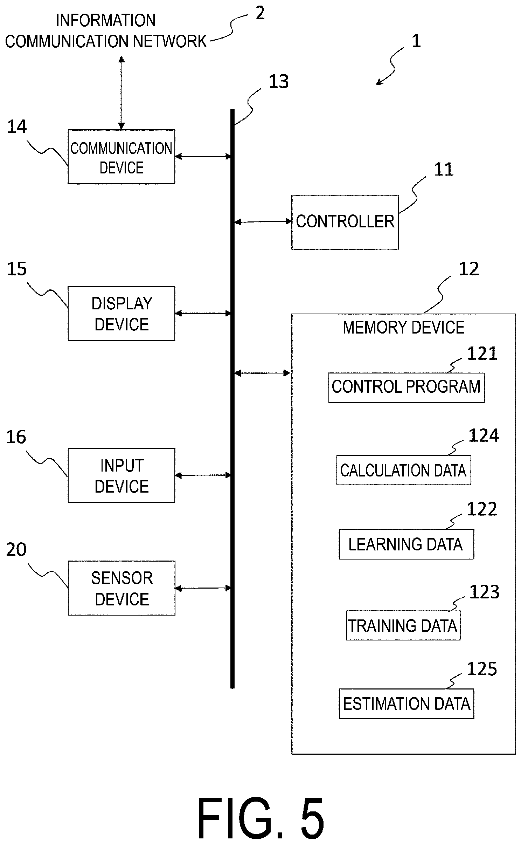

FIG. 5 is a schematic view illustrating the configuration of a sleep estimation system 1 according to another embodiment.

The sleep estimation system 1 according to the other embodiment further includes a sensor device 20 that acquires blood flow data. Specifically, the sleep estimation system 1 according to the other embodiment may further include the sensor device 20 including a light emitting unit that emits light to a target site of a user, and a light receiving unit that receives interference light including light that has scattered due to blood flow of the user. In addition, the controller 11 can acquire blood flow data on the basis of a frequency spectrum of the output from the light receiving unit. Thus, the sleep estimation system 1 can improve convenience.

Note that the sensor device 20 and the other device may be electrically or optically connected to each other through the bus 13. In addition, the sensor device 20 may be able to communicate with each of the devices in the sleep estimation system 1 through the information communication network 2. The information communication network 2 includes, for example, at least one of a wireless network and a wired network. The information communication network 2 includes, for example, a wireless local area network (LAN), the Internet, and the like.

The sensor device 20 according to an embodiment may be, for example, a laser-Doppler flowmeter. Although not illustrated, the laser-Doppler flowmeter includes a light emitting unit that emits light to a target site of a user, a light receiving unit that receives light, and a controller that acquires blood flow data.

In this case, the sensor device 20 can utilize the Doppler effect to measure the blood flow. The frequency of the scattering light, which scatters due to the flowing blood, shifts (Doppler shift) due to the Doppler effect. Light emitted from a typical fluorescent lamp or the like is light including light with various frequencies and various intensities. Thus, it is difficult to know a change in frequency resulting from the Doppler shift. On the other hand, since laser light is light that contains a predominant amount of light having a specific frequency, it is easy to observe the change in frequency due to the Doppler shift. Thus, the sensor device 20 can utilize interference light including scattering light to measure the blood flow.

First, the sensor device 20 acquires a beat signal (also referred to as a beat signal) occurring as a result of interference of light between scattering light from a substance at rest and scattering light from a moving substance, by using the Doppler effect. Note that the beat signal is a relationship between intensity and time. Next, by performing a Fourier transform on the beat signal, the sensor device 20 can acquire, as a frequency spectrum, a relationship between the frequency and the intensity of the output from the light receiving unit. Here, the frequency and the frequency intensity of the output from the light receiving unit depend on the Doppler effect. That is, the frequency spectrum changes in accordance with the amount of flow or the flow rate of the blood. Thus, the sensor device 20 can compute the blood flow data on the basis of the frequency spectrum.

Furthermore, the frequency spectrum has a frequency range in which the intensity tends to decrease with changes in flow (e.g., increase in flow rate or velocity) and a frequency range in which the intensity tends to increase. Thus, the controller of the sensor device 20 may select a frequency band suitable for acquiring the blood flow data from among the frequencies in the acquired frequency spectrum, thereby acquiring the blood flow data. Thus, the sensor device 20 can improve the accuracy of the blood flow data included in the learning data 122, the training data 123, and the estimation data 125. That is, the sleep estimation system 1 can improve the accuracy of estimation of a sleep state.

The blood flow data included in the learning data 122, the training data 123, and the estimation data 125 may be left as a frequency spectrum. In this case, the frequency spectrum may be data that has undergone an arbitrary transformation. Specifically, the sleep estimation system 1 may use, as the blood flow data, data acquired by wavelet transforming the frequency spectrum (transformed data). With the wavelet transformation, the frequency spectrum is weighted by the measurement time for each frequency component. That is, the transformed data is data including a change in the frequency spectrum over time. Thus, since the features to be learned increase, the sleep estimation system 1 can improve the accuracy of estimation of a sleep state.

As one example, description will be made of a case where the sensor device 20 computes the coefficient of variation of vasomotion as the blood flow data. First, the sensor device 20 performs a Fourier transform on the beat signal (first beat signal) to acquire a frequency spectrum. Next, the sensor device 20 applies filtering to the frequency spectrum so that only the frequency range including a component of vasomotion is left. Then, the sensor device 20 performs a Fourier inverse transform on the filtered frequency spectrum to acquire the beat signal (second beat signal) again. The sensor device 20 can compute the coefficient of variation of the intensity of this second beat signal as the coefficient of variation of vasomotion. The sleep estimation system 1 according to an embodiment can set the computed coefficient of variation of vasomotion as the blood flow data used as the learning data 122 or the estimation data 125.

In this manner, the sleep estimation system 1 has been described in detail. However, the description above is given merely as an example in all aspects, and the present disclosure should not be limited to this. For example, the neural network may be a convolutional neural network (Convolutional Neural Network: CNN), a recurrent neural network (Recurrent Neural Network: RNN), a long short term memory (LSTM), or the like. In addition, the learning model of the approximator 17 is not limited to that described in the embodiments described above, provided that the sleep estimation system 1 can estimate a sleep state of a user on the basis of the learning data 122. Note that the various types of examples described above can be combined and applied as long as they do not contradict each other. In addition, it should be understood that a large number of examples that have not been given here can also be conceived to fall within the scope of the present disclosure.

The sleep estimation system 1 according to an embodiment includes: the input device 16 configured to input the blood flow data (estimation data 125) on a user; an approximator 17 configured to calculate the output data 19 indicating a sleep state of the user on the basis of the blood flow data; and the controller 11 configured to transmit the blood flow data to the approximator and perform processing of the calculation. In the sleep estimation system 1, the approximator is subjected to a learning process using the learning data 122 including data of the same type as the blood flow data and the training data 123 including data of the same type as the output data associated with the learning data.

### Sleep Estimation Device

The sleep estimation system 1 described in the embodiments described above may be configured as one device (sleep estimation device) having each device as a functional unit. A sleep estimation device according to one embodiment includes: an input unit (for example, the communication device 14, the input device 16, and the like) configured to receive blood flow data on a user; and a control unit (for example, the controller 11) that constitutes an approximator (for example, the approximator 17) including an input layer in which blood flow data is input, a hidden layer in which calculation based on a learned parameter is performed on an output from the input layer, and an output layer in which a calculation result of the hidden layer is output as a sleep state of the user.

The sleep estimation device described above may further include a sensor unit (for example, the sensor device 20) including a light emitting unit that emits light to a target site of a user, and a light receiving unit that receives interference light including light that has scattered due to blood flow of the user. In addition, the control unit may acquire blood flow data on the basis of a frequency spectrum of an output from the light receiving unit.

### Method of Estimating Sleep

Each of the steps performed by the control program 121 that the sleep estimation system 1 described in the above embodiment has may be interpreted as the invention of a method of estimating sleep. A method of estimating sleep according to an embodiment is a method of estimating a sleep state of a user by using an approximator including: an input layer configured to receive blood flow data of a user; a hidden layer configured to perform calculation based on a learned parameter to an output from the input layer; and an output layer configured to output a calculation result of the hidden layer, as a sleep state of a user. A method of estimating sleep includes the steps of: transmitting, by the input layer, the blood flow data inputted into the approximator to the hidden layer; performing, by the hidden layer, calculation based on a learned parameter on the blood flow data; and outputting, by the output layer, an estimated sleep state of the user on the basis of a calculation result of the hidden layer.

The method of estimating sleep may include a step (learning process) of preparing the approximator by using the learning data 122 and the training data 123.

The method of estimating sleep described above may further include the steps of: emitting, by a light emitting unit of the sensor device 20, light to a target site of the user; receiving, by a light receiving unit of the sensor device 20, interference light including light that has scattered due to a blood flow of the user; and acquiring, by the controller 11, blood flow data on the basis of a frequency spectrum of an output from the light receiving unit.

### Reference Signs List

- 1: Sleep estimation system (sleep estimation device)
- 11: Controller (control unit)
- 12: Memory device
- 121: Control program
- 122: Learning data
- 123: Training data
- 124: Calculation data
- 125: Estimation data
- 13: Bus
- 14: Communication device (input unit)
- 15: Display device
- 16: Input device (input unit)
- 17: Approximator
- 171: Input layer
- 172: Hidden layer
- 173: Output layer
- 19: Output data
- 20: Sensor device (sensor unit)
- 2: Information communication network

## Claims

1. A sleep estimation system comprising:
an input device configured to receive blood flow data on a user; and
a controller configured to calculate output data indicating a sleep state of the user from the blood flow data, wherein
the controller comprises an approximator capable of calculating the output data from the blood flow data.

2. The sleep estimation system according to claim 1, wherein
the blood flow data comprises data related to at least one of an amount of blood flow, a coefficient of variation of vasomotion, and a heart rate.

3. The sleep estimation system according to claim 1 or 2, wherein
the sleep state comprises states indicating wakefulness, REM sleep, and non-REM sleep.

4. The sleep estimation system according to claim 3, wherein
the non-REM sleep comprises non-REM sleep of stages 1 to 4.

5. The sleep estimation system according to any one of claims 1 to 4, wherein
the approximator comprises a hidden layer in which calculation based on a learned parameter is performed on an output from an input layer, and
the learned parameter is a parameter that weights a relationship between the blood flow data and the sleep state in the approximator.

6. The sleep estimation system according to any one of claim 5, wherein
the learned parameter is a parameter that weights a relationship between the blood flow data and the sleep state in the approximator for each sleep state.

7. The sleep estimation system according to any one of claims 1 to 6, wherein
a site where the blood flow data is acquired is an ear, a finger, a wrist, a forehead, a nose, or a neck.

8. The sleep estimation system according to any one of claims 1 to 7, further comprising:
a sensor device comprising:
a light emitting unit configured to emit light to a target site of the user; and
a light receiving unit configured to receive interference light including the light that has scattered due to blood flow of the user, wherein
the controller acquires the blood flow data on a basis of a frequency spectrum of an output from the light receiving unit.

9. The sleep estimation system according to claim 8, wherein
the controller selects a frequency band of the frequency spectrum to acquire the blood flow data.

10. The sleep estimation system according to claim 8 or 9, wherein
the blood flow data includes data in which the frequency spectrum is wavelet transformed.

11. A sleep estimation program for causing a computer to function as the controller provided in the sleep estimation system according to any one of claims 1 to 10.

12. A sleep estimation device comprising:
an input unit configured to receive blood flow data on a user; and
a control unit comprising an approximator comprising an input layer, configured to receive the blood flow data, a hidden layer configured to perform calculation based on a learned parameter on an output from the input layer, and an output layer configured to output a calculation result of the hidden layer as a sleep state of the user.

13. The sleep estimation device according to claim 12, further comprising:
a sensor unit comprising:
a light emitting unit configured to emit light to a target site of the user; and
a light receiving unit configured to receive interference light including the light that has scattered due to blood flow of the user, wherein
the control unit acquires the blood flow data on a basis of a frequency spectrum of an output from the light receiving unit.

14. A method of estimating sleep using an approximator comprising an input layer configured to receive blood flow data on a user; a hidden layer configured to perform calculation based on a learned parameter on an output from the input layer; and an output layer configured to output a calculation result of the hidden layer as a sleep state of the user, the method comprising the steps of:
transmitting, by the input layer, the blood flow data inputted into the approximator to the hidden layer;
performing, by the hidden layer, calculation based on a learned parameter on the blood flow data; and
outputting, by the output layer, an estimated sleep state of the user on a basis of a calculation result of the hidden layer.

15. The method of estimating sleep according to claim 14, wherein
the blood flow data inputted into the approximator is the blood flow data acquired by emitting, by a light emitting unit of a sensor device, light to a target site of the user, receiving, by a light receiving unit of the sensor device, interference light including the light that has scattered due to blood flow of the user, and using a frequency spectrum of an output from the light receiving unit.
